# EUROPÄISCHE PATENTSCHRIFT

(11) **EP 0 663 386 B1**
(45) Veröffentlichungstag und Bekanntmachung des Hinweises auf die Patenterteilung: **14.04.1999**
(21) Anmeldenummer: 95100110.6
(22) Anmeldetag: 05.01.1995
(51) Int. Cl.: C07C 67/03, C07C 69/54

(54) **Verfahren zur Umesterung von (Meth)acrylsäureestern**
Process for the transesterification of (meth-)acrylic esters
Procédé de transestérification d'esters (méth-)acryliques

(30) Priorität: 17.01.1994 DE 4401132
(43) Veröffentlichungstag der Anmeldung: 19.07.1995
(73) Patentinhaber: Röhm GmbH, 64293 Darmstadt (DE)
(72) Erfinder: Knebel, Joachim, Dr., D-64293 Darmstadt (DE); Pfirmann, Martina, Dr., D-64347 Griesheim (DE); Ohl, Thomas, D-64289 Darmstadt (DE)

(56) Entgegenhaltungen:
- EP-A- 0 646 567
- DE-C- 4 317 428
- DATABASE WPI Week 9115 Derwent Publications Ltd., London, GB; AN 91-104785 & JP-A-03 041 051 (IDEMITSU PETROCHEM KK) , 21.Februar 1991
- PATENT ABSTRACTS OF JAPAN vol. 8 no. 3 (C-203) ,7.Januar 1984 & JP-A-58 170730 (MITSUI TOATSU KAGAKU KK) 7.Oktober 1983,
- PATENT ABSTRACTS OF JAPAN vol. 3 no. 68 (C-048) ,13.Juni 1979 & JP-A-54 041814 (NITTO CHEM IND CO LTD) 3.April 1979,

## Beschreibung

Die Erfindung betrifft ein Verfahren zur Umesterung von (Meth)acrylsäureestern mit insbesondere mehrfunktionellen Alkanolen unter Verwendung eines Mischkatalysators bestehend aus Diorganylzinnoxid und Organylzinnhalogenid.

### Stand der Technik

Die Verwendung von Diorganylzinnoxiden bzw. von Organylzinnhalogeniden als Umesterungskatalysatoren ist bekannt. So beschreibt beispielsweise DE-AS 1005947, daß Di- und Triorganozinnverbindungen Veresterungs- und Umesterungsreaktionen von (Meth)acrylsäure bzw. (Meth)acrylsäureestern wirksam katalysieren. Die vorteilhaften Wirkungen der dort beschriebenen Katalysatoren umfassen hohe katalytische Wirksamkeit, geringe Neigung zur Dehydratisierung insbesondere sekundärer Alkohole sowie hohe Esterausbeuten.

Speziellere Anwendungen solcher Katalysatoren werden beispielsweise von DE-OS 1965308, DE-OS 2816516 oder EP-A 433 135 angeführt.

DE-OS 1965308 umfaßt ein Verfahren zur Herstellung von Dimethylaminoethylmethacrylat aus Dimethylaminoethanol und einem Alkylmetha-crylat unter Katalyse von Di-n-butyl-zinnoxid, wobei zunächst das ent-stehende Azeotrop aus Alkylmethacrylat und Alkanol und darauf folgend das Reaktionsprodukt Dimethylaminoethylmethacrylat abdestilliert werden.

In DE-OS 2816516 wird ein Verfahren zur Herstellung N-substituierter (Meth)acrylamide durch Aminolyse von Alkyl(meth)acrylaten mit aliphatischen oder aromatischen Aminen in Gegenwart von Dialkylzinnoxid-Katalysatoren beschrieben. Die Aminolyse der Estergruppe wird mit diesen Katalysatoren solchermaßen beschleunigt, daß die unerwünschten Michael-Addukte der Amine an die Doppelbindung der (Meth)acrylester nur in untergeordneten Mengen auftreten.

EP-A 433 135 beschreibt ein Verfahren zur Umsetzung von Alkyl(meth)-acrylaten mit Hydroxialkylimidazolidin-2-onen zu entsprechenden Um-esterungsprodukten, bei dem als Katalysatoren Dialkylzinnoxide, Dialkoxyl-zinnoxide und Dialkylzinndiester eingesetzt werden können. Ebenso wie bei DE-OS 2816516 wird die Bildung der unerwünschten Michael-Addukte praktisch vermieden.

JP-A 03 041 051 (Idemitsu) beschreibt die Herstellung von Estern der Methacrylsäure mit mehrwertigen Alkoholen, wie beispielsweise Glycerin oder Pentaerythrit. Als Katalysator wird ein Gemisch aus organischen Zinnsalzen und Bleioxiden verwendet.

JP 58 170 730 (Mitsui) beschreibt die Herstellung von Methacrylsäureestern aus Methacrylsäure und aromatischen Alkoholen in Gegenwart einer halogenhaltigen Organozinn-Verbindung

JP 54 041 814 (Nitto Chem. Ind) beschreibt die Umesterung von niederen Methacrylsäureestern mit höheren, auch mehrwertigen Alkoholen unter Katalyse von Zinnalkylen, wie beispielsweise Tributylzinmethoxid.

J. Otera et al. beschreiben die Herstellung von Tetraorganodistannoxanen aus Diorganylzinnoxiden und Diorganylzinndichlorid (J. Org. Chem. 56 (18), Seiten 5307 bis 5311 (1991) und Chem. Rev. 93, Seiten 1449 bis 1470 (1993)). Solche Tetraorganodistannoxane werden als besonders effektive Umesterungskatalysatoren beschrieben.

### Aufgabe und Lösung

Die Präparation von mono- oder mehrfunktionellen (Meth)acrylsäureestern durch Umesterung von (Meth)acrylsäureestern, welche kleine Esterreste, beispielsweise Alkylreste, aufweisen, mit mono- oder mehrfunktionellen Alkoholen unter Katalyse von Diorganylzinnoxiden oder Diorganylzinnhalogeniden nach den Verfahren des Standes der Technik, verläuft oftmals mit unbefriedigend geringen Umsätzen der Edukte in akzeptablen Reaktionszeiten. Dies ist insbesondere der Fall bei der Umsetzung von (Meth)acrylsäureestern mit mehrfunktionellen Alkoholen.

Überraschenderweise wurde gefunden, daß solche Umesterungsreaktionen von (Meth)acrylsäureestern mit mono- oder mehrfunktionellen Alkoholen besonders vorteilhaft in Gegenwart von Mischkatalysatoren aus Diorganylzinnoxid und Organylzinnhalogenid durchgeführt werden können. Dabei ist die von Otera et al. beschriebene Herstellung von Tetraorganodistannoxan als katalytische wirksame Substanz aus Diorganozinnoxid und Diorganozinndihalogenid nicht notwendig.

Im erfindungsgemäßen Verfahren werden (Meth)acrylsäureester der Formel I 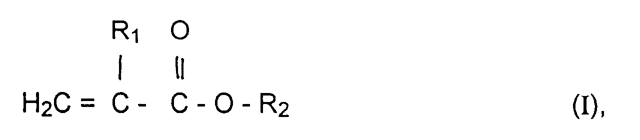 wobei R₁ für H oder CH₃ und R₂ für einen Alkylrest 1 bis 6 Kohlenstoffatomen stehen, mit davon verschiedenen Alkoholen, die eine oder mehrere Hydroxylgruppen aufweisen, in Gegenwart von 0,01 bis 10 Gew.-%, insbesondere von 0,1 bis 5 Gew.-% bezogen auf das gesamte Reaktionsgemisch, eines Mischkatalysators, umgesetzt, bestehend zu:

5 bis 95 Mol-% bezogen auf den gesamten Mischkatalysator aus Diorganylzinnoxiden der Forme II. 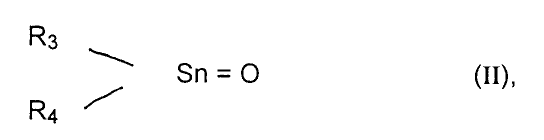 wobei R₃ und R₄ für aliphatische, aromatische oder araliphatische Reste mit 1 bis 12 Kohlenstoffen stehen, sowie zu 95 bis 5 Mol-% bezogen auf den gesamten Mischkatalysator aus Organylzinnhalogeniden der Formeln IIIa und IIIb 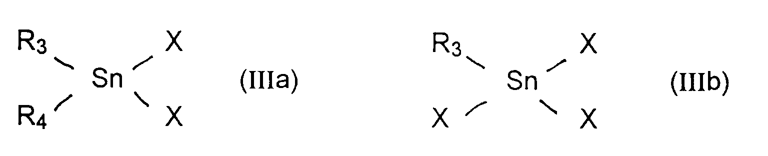
wobei R₃ und R₄ wiederum für aliphatische, aromatische Reste mit 1 bis 12 Kohlenstoffatomen stehen, sowie X für Cl, Br, CN, NCO oder NCS steht, und
wobei sich die Diorganozinnoxide II und die Organozinnhalogenide IIIa und IIIb zu 100 Mol-% addieren.

Besonders bevorzugt liegen die Anteile der Diorganozinnoxide II zwischen 25 und 75 Mol-% und die Anteile der Organozinnhalogenide IIIa und IIIb zwischen 75 und 25 Mol-%.

Bevorzugt weisen die bei der Umesterung verwendeten Alkohole zwei oder mehrere veresterbare Hydroxylgruppen auf. Besonders bevorzugt eingesetzt werden difunktionelle Alkohole der Formel IV:

HO - R' - OH (IV),

wobei R' für einen gegebenenfalls verzweigten aliphatischen oder araliphatischen Rest mit 2 bis 24 Kohlenstoffatomen steht,
trifunktionelle Alkohole der Formel V: 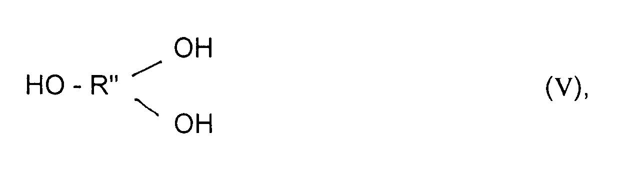
wobei R' für einen gegebenenfalls verzweigten aliphatischen oder araliphatischen Rest mit 3 bis 30 Kohlenstoffatomen steht,
sowie tetrafunktionelle Alkohole der Formel VI: 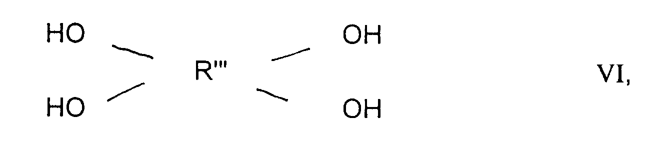
wobei R''' für einen gegebenenfalls verzweigten aliphatischen oder araliphatischen Rest mit 4 bis 40 Kohlenstoffatomen steht,
und das der Mischkatalysator nach Beendigung der Umesterungsreaktion durch Zugabe von Wasser in Mengen von 10 - 150 Gew.-Teilen bezogen auf 100 Gew.-Teile des gesamten Reaktionsgemischs abgetrennt wird.

### Durchführung der Erfindung

### Die Edukte und Katalysatoren

Als Acryl- oder Methacrylsäureester der Formel I können beispielsweise eingesetzt werden: Ethyl(meth)acrylat, n-Propyl(meth)acrylat, i-Propyl(meth)acrylat, n-Butylmethacrylat, i-Butyl(meth)acrylat, tert.-Butyl(meth)acrylat, n-Pentyl(meth)arylat, n-Hexyl(meth)acrylat, oder bevorzugt Methylmethacrylat sowie Butylacrylat.
Die für die Umesterung eingesetzten Alkohole können eine oder mehrere zur Umesterung befähigte Hydroxylgruppen aufweisen, in anderen Worten mono- oder mehrfunktionell sein.
Beispielhaft für viele seien folgende monofunktionelle Alkohole genannt: Ethanol, n-Propanol, i-Propanol, n-Butanol, tert.-Butanol, n-Pentanol, i-Pentanol, tert.-Pentanol, n-Hexanol, n-Octanol, 2-Ethylhexan-1-ol, n-Decanol, n-Tetradecanol, n-Eicosanol, Cyclohexanol, 3,3,5-Trimethylcyclohexan-1-ol, Tetrahydrofurfurylalkohol, 2-Phenylethanol. Für die difunktionellen Alkohole der Formel IV als Repräsentanten der bevorzugten Alkohole mit zwei oder mehreren Hydroxylgruppen werden folgende Beispiele angeführt: Ethylenglykol, Diethylenglykol, Triethylenglykol, Tetraethylenglykol, 1,3-Propandiol, 1,2-Propandiol, 1,3-Butandiol, 1,4-Butandiol, 1,5-Pentandiol, 1,6-Hexandiol, 1,8-Octandiol, 1,10-Decandiol, 1,12-Docecandiol, Cyclohexandiol-1,4.
Trifunktionelle Alkohole der Formel V bzw. tetrafunktionelle Alkohole der Formel VI sind beispielsweise: Trimethylolethan, Trimethylolpropan, 1,2,4-Butantriol, 1,2,6-Hexantriol, Pentaerythritol, Erythritol, Threitol.
Als Vertreter von penta- und hexafunktionellen Alkoholen, die ebenfalls als Umesterungskomponenten eingesetzt werden können, seien beispielhaft genannt: Arabinitol, Ribitol, Xylitol, Sorbitol, Glucitol und Mannitol, alle auch als Zuckeralkohole bekannt (vgl. z. B. Kirk-Othmer, Encyclopedia of Chemical Technology, 3rd Ed., Vol. 1, Seiten 754 bis 789, John Wiley, New York, 1978).

Die Umsetzung von Acryl- oder Methacrylestern der Formel I mit den mono- oder mehrfunktionellen Alkoholen wird in Gegenwart von 0,01 bis 10 Gew.-% bevorzugt von 0,1 bis 5 Gew.-%, besonders bevorzugt von 0,2 bis 2 Gew.-% bezogen auf die gesamte Reaktionsmischung, eines Mischkatalysators bestehend aus 5 bis 95 Mol-%, vorzugsweise 25 bis 75 Mol-%, bezogen auf den Mischkatalysator aus Diorganylzinnoxid der Formel II und 95 bis 5 Mol-%, vorzugsweise 75 bis 25 Mol-% aus Organylzinnhalogenid der Formel IIIa und/oder IIIb.

Beispielhaft für Diorganylzinnoxide der Formel II seien genannt: Dimethylzinnoxid, Diethylzinnoxid, Dipropylzinnoxid, Dihexylzinnoxid, Dicyclohexylzinnoxid, Didodecylzinnoxid, Diphenylzinnoxid, Dibenzylzinnoxid, sowie bevorzugt Dibutylzinnoxid und Dioctylzinnoxid.

Als Beispiele für Diorganylzinnhalogenid der Formel IIIa seien aufgeführt: Dimethylzinndichlorid, Dimethyldizinnbromid, Diethylzinndichlorid, Dibutylzinndibromid, Dihexylzinndichlorid, Dihexylzinndiiodid, Dioctylzinndibromid, Diphenylzinndichlorid, Dibenzylzinndichlorid, sowie bevorzugt Dibutylzinndichlorid und Dioctylzinndichlorid.

Beispielhaft für Organozinntrihalogenide seien genannt: Methylzinntrichlorid, Methylzinntribromid, Ethylzinntrichlorid, Butylzinntribromid, Butylzinntriiodid, Cyclohexylzinntrichlorid, Phenylzinntrichlorid, Phenylzinntribromid, Benzylzinntrichlorid sowie bevorzugt Butylzinntrichlorid oder Octylzinntrichlorid.

### Das Verfahren

Die Umsetzung von Acryl- oder Methacrylsäureestern der Formel I mit den mono- oder mehrfunktionellen Alkoholen wird bei Temperaturen zwischen 30 und 180 °C, vorzugsweise zwischen 50 und 130 Grad in Gegenwart von 0,01 bis 10 Gew.-%, bevorzugt von 0,1 bis 5 Gew.-%, besonders bevorzugt von 0,2 bis 2 Gew.-%, bezogen auf die gesamte Reaktionsmischung des Mischkatalysators aus Diorganylzinnoxid und Organylzinnhalogenid durchgeführt.

Formal reagieren äquimolare Mengen an (Meth)acrylsäureestern der Formel I und Hydroxylgruppen der mono- oder mehrfunktionellen Alkohole zu den gewünschten Endprodukten. In der Praxis ist es jedoch zweckmäßig, während der Umsetzung die (Meth)acrylsäureestern I im Überschuß zu halten, wobei die (Meth)acrylsäure I in Mengen von 1,2 bis 15 Mol, vorzugsweise 2 bis 10 Mol, pro Mol Hydroxylgruppen eingesetzt werden.

Zur Vermeidung von durch Polymerisation der (Meth)acrylsäureestern verursachten Ausbeuteverlusten ist es zweckmäßig, die Umsetzung und Aufarbeitung des Reaktionsgemisches in Gegenwart von Polymerisationsinhibitoren durchzuführen, wie beispielsweise gelösten Sauerstoff, Phenothiazin oder vorzugsweise Hydrochinonmonomethylether.

Die Umsetzung kann unter Normaldruck, Unter- oder Überdruck durchgeführt werden, wobei die Reaktionsführung diskontinuierlich oder kontinuierlich erfolgen kann. Im allgemeinen werden die Edukte, (Meth)acrylsäureester I und mono- oder mehrfunktioneller Alkohol, gemeinsam in Gegenwart des Mischkatalysators auf Reaktionstemperatur erhitzt und dabei kontinuierlich der abgespaltene Alkohol R₂OH sowie der überschüssige (Meth)acrylsäureester I, vorzugsweise gemeinsam in einem Azeotrop, abdestilliert. Die Reaktionszeiten liegen im allgemeinen zwischen 1 und 20 Stunden, vorzugsweise zwischen 2 und 8 Stunden, und hängen von der Reaktionstemperatur bzw. von der eingesetzten Menge des Katalysatorgemisches ab. Weiterhin ist es möglich, die Umsetzung in Gegenwart eines inerten Lösungsmittels, wie beispielsweise Toluol oder Cyclohexan, durchzuführen.
Mach Beendigung der Umsetzung wird der überschüsssige (Meth)acrylsäureester I teilweise oder vorzugsweise vollständig, beispielsweise durch Abdestillieren, aus dem Reaktionsprodukt entfernt.
Die Abtrennung des Mischkatalysators wird durch Zugabe von Wasser zum Reaktionsgemisch, das den überschüssigen (Meth)acrylsäureester I noch teilweise enthalten kann, bewerkstelligt. Dazu wird Wasser in Mengen von 1 bis 200 Gew.-Teilen, vorzugsweise in Mengen von 10 bis 150 Gew.-Teilen, besonders bevorzugt in Mengen von 20 bis 100 Gew.-Teilen bezogen auf 100 Gew.-Teile des erhaltenen Reaktionsgemisches, vorzugsweise nach dessen Abkühlen, insbesondere bei Temperaturen zwischen 10 und 50 °C, zugesetzt. Dabei fällt der Mischkatalysator in filtrierbarer Form an.

Das erfindungsgemäße Verfahren zur Herstellung von (Meth)acrylsäureestern durch Umesterung von (Meth)acrylsäureestern I mit mono- oder mehrfunktionellen Alkoholen liefert insbesondere bei der Herstellung mehrfunktioneller (Meth)acrylsäureester deutlich höhere Ausbeuten und deutlich weniger Nebenprodukte als die Verfahren des Standes der Technik.

Die folgenden Beispiele sollen die Erfindung erläutern.

### BEISPIELE

### Beispiel 1: Triethyhlenglycoldimethacrylat

In einen 2-Liter-Vierhalskolben mit Thermometer, mechanischer Rührung, Lufteinleitrohr und aufgesetzter Füllkörperkolonne (Durchmesser 3 cm, Länge 40 cm) sowie Kolonnenkopf mit Rücklaufteiler gibt man 240 g Triethylenglycol (1,6 Mol), 880 g Methylmethacrylat (8,8 Mol), 5 g Dibutylzinnoxid (0,02 Mol) und 6,1 g Dibutylzinndichlorid (0,02 Mol) als Mischkatalysator sowie 0,046 g Hydrochinonmonomethylether als Polymerisationsinhibitor. Unter langsamem Lufteinleiten erhitzt man die Reaktionsmischung auf eine Sumpftemperatur von 100 - 114 °C, wobei bei einem Rücklaufverhältnis von 1 : 1 bis 5 : 1 ein Methanol-Methylmethacrylatgemisch abgezogen wird. Dabei stellt sich eine Kopftemperatur von anfänglich 70 °C ein, die allmählich ansteigt. Nach 2,5 Stunden beträgt sie konstant 100 °C und die Umesterung ist beendet. Man zieht überschüssiges Methylmethacrylat im Vakuum ab und erhält als Rückstand 400 g (87 % Ausbeute) Triethylenglycoldimethylacrylat mit einer gaschromatographischen Zusammensetzung wie folgt:
- 97,5 %: Triethylenglycoldimethacrylat
- 0,8 %: Triethylenglycolmonomethacrylat
- 1,3 %: Methylmethacrylat

### Beispiel 2: Triethylenglycoldimethacrylat

Durchführung wie in Beispiel 1 beschrieben, jedoch unter Verwendung von 7,1 g Dioctylzinnoxid (0,02 Mol) und 4,1 g Dioctylzinndichlorid (0,01 Mol) als Mischkatalysator. Man erhält 407 g (89 % Ausbeute) Triethylenglycoldimethacrylat einer gaschromatographischen Zusammensetzung wie folgt:
- 97,2 %: Triethylenglycoldimethacrylat
- 1,3 %: Triethylenglycolmonomethacrylat
- 1,3 %: Methylmethacrylat

### Beispiel 3: Triethylenglycoldimethacrylat

Durchführung wie in Beispiel 1 beschrieben, jedoch unter Verwendung von 5 g (0,02 Mol) Dibutylzinnoxid und 6 g (0,021 Mol) Butylzinntrichlorid als Mischkatalysator. Nach 3,5 Stunden Reaktionszeit ist die Umesterung beendet und das überschüssige Methylmethacrylat wird in Vakuum abgezogen. Als Rückstand werden 422 g (92 % Ausbeute) Triethylenglycoldimethacrylat einer gaschromatographischen Zusammensetzung wie folgt erhalten:
- 98,2 %: Triethylenglycoldimethacrylat
- 0,9 %: Triethylenglycolmonomethacrylat
- 0,5 %: Methylmethacrylat

### Vergleichsbeispiel 1:

Herstellung von Triethylenglycoldimethacrylat unter alleiniger Verwendung von Dibutylzinndichlorid als Katalysator.

Durchführung wie in Beispiel 1 beschrieben, jedoch unter Verwendung von 11,2 g (0,037 Mol) Dibutylzinndichlorid als alleinigem Katalysator. Nach 3 Stunden Reaktionszeit hat sich noch kein Methanol gebildet, d. h. es findet keine Umesterung statt.

### Beispiel 4: Ethylenglycoldimethacrylat

Durchführung wie in Beispiel 1 beschrieben, jedoch unter Verwendung von 173 g (2,8 Mol) Ethylenglycol, 1200 g (12 Mol) Methylmethacrylat, 6,4 g (0,018 Mol) Dioctylzinnoxid und 7,3 g (0,018 Mol) Dioctylzinndichlorid als Mischkatalysator sowie 0,084 g Hydrochinonmonomethylether. Nach 6 Stunden Reaktionszeit ist die Umesterung beendet und das überschüssige Methylmethacrylat wird im Vakuum abgezogen. Als Rückstand werden 437 g (79 % Ausbeute) Ethylenglycoldimethacrylat einer gaschromatographischen Zusammensetzung wie folgt erhalten:
- 98,0 %: Ethylenglycoldimethacrylat
- 1,5 %: Ethylenglycolmonomethacrylat
- 0,4 %: Methylmethacrylat

### Vergleichsbeispiel 2

Herstellung von Ethylenglycoldimethacrylat unter alleiniger Verwendung von Dioctylzinnoxid als Katalysator.

Durchführung wie in Beispiel 1 beschrieben, jedoch unter Verwendung von 248 g (4,0 Mol) Ethylenglycol, 900 g (9 Mol) Methylmethacrylat, 11,5 g ( 0,032 Mol) Dioctylzinnoxid als Katalysator sowie 0,23 g Hydrochinonmonomethylether. Nach 6,5 Stunden Reaktionszeit wird das zweiphasige Reaktionsgemisch abgekühlt und gaschromatographisch analysiert.
Man erhält folgendes Ergebnis:

### Obere Phase

- 1,5 %: Ethylenglycoldimethacrylat
- 12,7 %: Ethylenglycolmonomethacrylat
- 4,3 %: Ethylenglycol
- 81,3 %: Methylmethacrylat

### Untere Phase

- 0,2 %: Ethylenglycoldimethacrylat
- 15,7 %: Ethylenglycolmonomethacrylat
- 63,0 %: Ethylenglycol
- 20,8 %: Methylmethacrylat

### Vergleichsbeispiel 3:

Herstellung von Ethylenglycoldimethacrylat unter alleiniger Verwendung von Butylzinntrichlorid als Katalysator.

Durchführung wie in Beispiel 1 beschrieben, jedoch unter Verwendung von 124 g (2,0 Mol) Ethylenglycol, 450 g (4,5 Mol) Methylmethacrylat, 5,7 g (0,02 Mol)

Butylzinntrichlorid als Katalysator und 0,12 g Hydrochinonmonomethylether. Nach 4 Stunden Reaktionszeit ist die Umesterung beendet. Die gaschromatographische Analyse des Reaktionsprodukts zeigt folgende Zusammensetzung:
- 5,9 %: Ethylenglycoldimethacrylat
- 28,5 %: Ethylenglycolmonomethacrylat
- 11,0 %: Ethylenglycol
- 52,9 %: Methylmethacrylat

### Beispiel 5: Pentaerythrittetramethacrylat

Durchführung wie in Beispiel 1 beschrieben, jedoch unter Verwendung von 136 g (1,0 Mol) Pentaerythrit, 1000 g (10,0 Mol) Methylmethacrylat, 5,0 g (0,02 Mol) Dibutylzinnoxid und 6,1 g (0,02 Mol) Dibutylzinndichlorid als Mischkatalysator sowie 0,57 g Hydrochinonmonomethylether. Nach 14 Stunden Reaktionszeit wird das überschüssige Methylmethacrylat im Vakuum entfernt. Die H-NMR-spektroskopische Analyse des Reaktionsprodukts zeigt folgende Zusammensetzung:
- 78 Mol-%: Pentaerythrittetramethacrylat
- 18 Mol-%: Pentaerythrittrimethacrylat
- 3 Mol-%: Pentaerythritdimethacrylat
- 1 Mol-%: Pentaerythritmonomethacrylat

### Vergleichsbeispiel 4:

Herstellung von Pentaerythrittetramethacrylat unter alleiniger Verwendung von Dibutylzinndichlorid als Katalysator.

Durchführung wie in Beispiel 5 beschrieben, jedoch unter Verwendung von 11,4 g (0,038 Mol) Dibutylzinndichlorid als Katalysator. Nach 3,5 Stunden Reaktionszeit hat sich noch kein Methanol gebildet, d. h. es findet keine Umesterung statt.

### Beispiel 6: Pentaerythrittetraacrylat

Durchführung wie in Beispiel 3 beschriebe, jedoch unter Verwendung von 136 g (1,0 Mol) Pentaerythrit, 1262 g (10,0 Mol) Butylacrylat, 6,3 (0,025 Mol) Dibutylzinnoxid und 7,7 g (0,025 Mol) Dibutylzinndichlorid als Mischkatalysator sowie 0,7 g Hydrochinonmonomethylether und 0,14 g Phenothiazin als Polymerisationsinhibitoren. Die Umsetzung wird bei einem Druck von 295 mbar durchgeführt. Nach 8 Stunden Reaktionszeit wird das überschüssige Butylacrylat im Vakuum entfernt. Die 1H-NMR-spektroskopische Analyse des Reaktionsprodukts zeigt folgende Zusammensetzung:
- 28 Mol-%: Pentaerythrittetraacrylat
- 48 Mol%: Pentaerythrittriacrylat
- 21 Mol-%: Pentaerythritdiacrylat
- 3 Mol-%: Pentaerythritmonoacrylat

### Beispiel 7: Tetrahydrofurfurylmethacrylat

Durchführung wie in Beispiel 1 beschrieben, jedoch unter Verwendung von 204 g (2,0 Mol) Tetrahydrofurfurylalkohol, 500 g (5,0 Mol) Methylmethacrylat, 4,4 g (0,0175 Mol) Dibutylzinnoxid und 2,7 g (0,008 Mol) Dibutylzinndichlorid als Mischkatalysator sowie 0,35 g Hydrochinonmonomethylether. Nach 2,5 Stunden Reaktionszeit ist die Umesterung beendet und das überschüssige Methylmethacrylat wird im Vakuum abgezogen. Als Rückstand werden 238 g (70 % Ausbeute) Tetrahydrofurfurylmethacrylat einer gaschromatographischen Zusammensetzung wie folgt erhalten:
- 94,7 %: Tetrahydrofurfurylmethacrylat
- 1,5 %: Tetrahydrofurfurylalkohol
- 2,7 %: Methylmethacrylat

## Patentansprüche

1. Verfahren zur Herstellung von Acryl- und Methacrylsäureestern durch Umsetzung von (Meth)acrylsäureestern der Formel I: 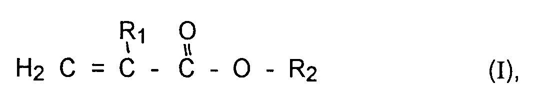 wobei
R₁ für H oder CH₃ und
R₂ für einen Alkylrest mit 1 bis 6 Kohlenstoffatomen stehen,
mit Alkoholen, die eine oder mehrere veresterbare Hydroxylgruppen aufweisen,
dadurch kennzeichnet,
daß als Umesterungskatalysator 0,01 bis 10 Gew.-% bezogen auf das gesamte Reaktionsgemisch Mischkatalysatoren eingesetzt werden, bestehend aus:
5 bis 95 Mol-% bezogen auf den Mischkatalysator an Diorganylzinnoxiden der Formel II: 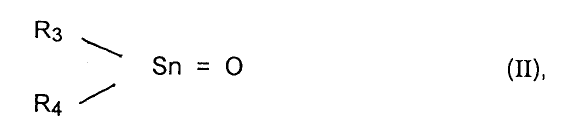 wobei
R₃ und R₄ für aliphatische, aromatische oder araliphatische Reste mit 1 bis 12 Kohlenstoffatomen stehen,
sowie aus
95 bis 5 Mol-% bezogen auf den Mischkatalysator an Organylzinnhalogeniden der Formel IIIa und/oder der Formel IIIb: 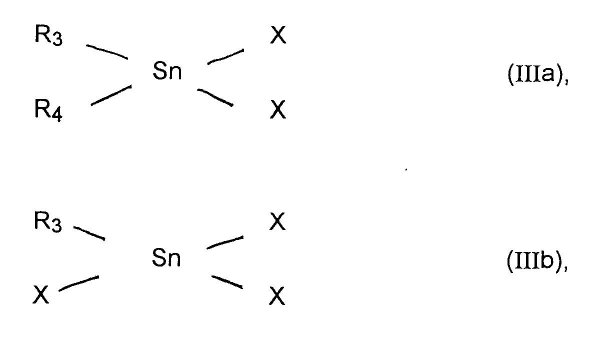 wobei
R3 und R4 wiederum für aliphatische, aromatische oder araliphatische Reste mit 1 bis 12 Kohlenstoffatomen stehen, sowie X für C1, Br, I, CN, NCO oder NCS steht und das der
Mischkatalysator nach Beendigung der Umesterungsreaktion durch Zugabe von Wasser in Mengen von 10 - 150 Gew.-Teilen bezogen auf 100 Gew.-Teile des gesamten Reaktionsgemischs abgetrennt wird.

2. Verfahren gemäß Anspruch 1, dadurch gekennzeichnet, daß die Mischkatalysatoren in Mengen von 0,1 bis 5 Gew.-% bezogen auf das gesamte Reaktionsgemisch eingesetzt werden.

3. Verfahren gemäß den Ansprüchen 1 und 2, dadurch gekennzeichnet, daß die Mischkatalysatoren aus 25 bis 75 Mol-% aus Diorganylzinnoxiden der Formel II und aus 75 bis 25 Mol-% aus Organylzinnhalogeniden der Formel IIIa und/oder Formel IIIb bestehen.

4. Verfahren gemäß den Ansprüchen 1 bis 3, dadurch gekennzeichnet, daß die bei der Umesterung eingesetzten Alkohole zwei oder mehrere veresterbare Hydroxylgruppen aufweisen.

5. Verfahren gemäß Anspruch 4, dadurch gekennzeichnet, daß die Alkohole mit mehreren veresterbaren Hydroxylgruppen ausgewählt sind aus der Gruppe:
difunktionelle Alkohole der Formel IV:
HO - R' - OH (IV),
wobei:
R' für einen gegebenenfalls verzweigten aliphatischen oder araliphatischen Rest mit 2 bis 24 Kohlenstoffatomen steht,
trifunktionelle Alkohole der Formel V: 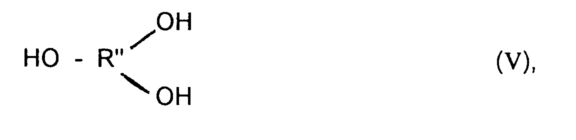
wobei: R'' für einen gegebenenfalls verzweigten aliphatischen oder araliphatischen Rest mit 3 bis 30 Kohlenstoffatomen steht,
sowie tetrafunktionelle Alkohole der Formel VI: 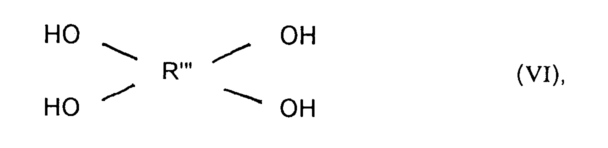 wobei:
R''' für einen gegebenenfalls verzweigten aliphatischen oder araliphatischen Rest mit 4 bis 40 Kohlenstoffatomen steht.

6. Verfahren gemäß den Ansprüchen 1 bis 5, dadurch gekennzeichnet, daß der (Meth)acrylsäureester der Formel I in Mengen von 1,2 bis 15 Mol pro Mol veresterbare Hydroxylgruppen eingesetzt wird.

7. Verfahren gemäß den Ansprüchen 1 bis 6, dadurch gekennzeichnet, daß die Umesterungsaktion bei Temperaturen zwischen 50 und 130 °C durchgeführt wird.

## Claims

1. A process for the preparation of acrylates and methacrylates by transesterification of (meth)acrylates of formula I: 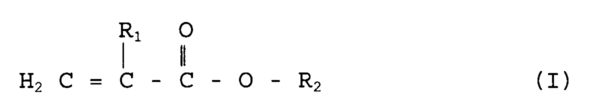 wherein
R₁ denotes H or CH₃ and
R₂ denotes an alkyl group with 1 to 6 carbon atoms,
with alcohols which have one or more esterifiable hydroxyl groups,
characterised in that
0.01 to 10% by weight of mixed catalysts, based on the total reaction mixture, are used as transesterification catalyst, the mixed catalyst comprising:
5 to 95 Mol%, based on the mixed catalyst, of diorganotin oxides of formula II: 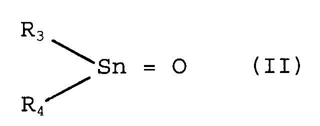 wherein
R₃ and R₄ denote aliphatic, aromatic or araliphatic groups with 1 to 12 carbon atoms,
and
95 to 5 Mol%, based on the mixed catalyst, of organotin halides of formula IIIa and/or formula IIIb: 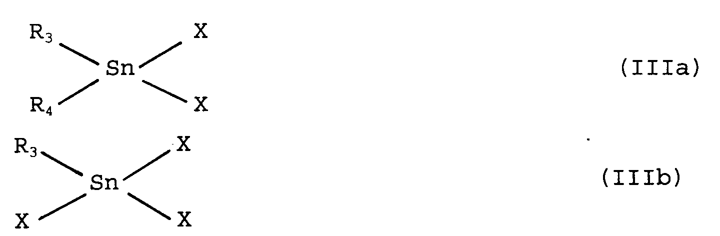 wherein
R₃ and R₄ again denote aliphatic, aromatic or araliphatic groups with 1 to 12 carbon atoms, and where X denotes Cl, Br, I, CN, NCO or NCS, and that the
mixed catalyst is separated off after conclusion of the transesterification by the addition of water in quantities of 10 to 150 parts by weight, based on 100 parts by weight of the total reaction mixture.

2. A process according to claim 1, characterised in that the mixed catalysts are used in quantities of 0.1 to 5% by weight, based on the total reaction mixture.

3. A process according to claims 1 and 2, characterised in that the mixed catalyst comprises 25 to 75 Mol% diorganotin oxides of formula II and 75 to 25 Mol% organotin halides of formula IIIa and/or formula IIIb.

4. A process according to claims 1 to 3, characterised in that the alcohols used for the transesterification have two or more esterifiable hydroxyl groups.

5. A process according to claim 4, characterised in that the alcohols with several esterifiable hydroxyl groups are selected from the group: difunctional alcohols of formula IV:
HO - R' - OH (IV)
wherein
R' denotes an optionally branched aliphatic or araliphatic group with 2 to 24 carbon atoms,
trifunctional alcohols of formula V: 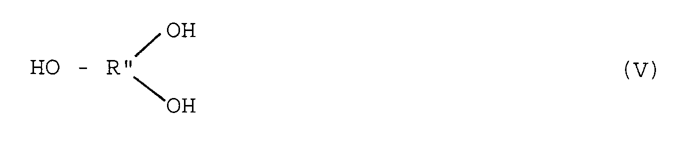 wherein:
R'' denotes an optionally branched aliphatic or araliphatic group with 3 to 30 carbon atoms,
and tetrafunctional alcohols of formula VI: 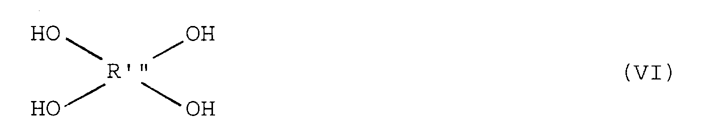 wherein:
R''' denotes an optionally branched aliphatic or araliphatic group with 4 to 40 carbon atoms.

6. A process according to claims 1 to 5, characterised in that the (meth)acrylate of formula I is used in quantities of 1.2 to 15 Mol/Mol of esterifiable hydroxyl groups.

7. A process according to claims 1 to 6, characterised in that the transesterification takes place at temperatures of between 50 and 130°C.

## Revendications

1. Procédé de fabrication d'esters d'acide acrylique et (méth)acrylique par réaction d'esters d'acide (méth)acrylique de formule I 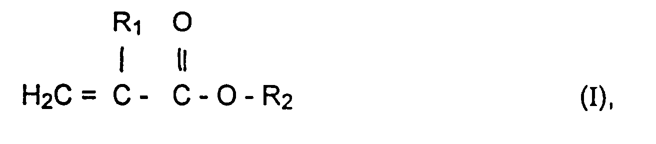 dans laquelle
R₁ représente H ou CH₃ et
R₂ représente un radical alkyle comportant de 1 à 6 atomes de carbone,
avec des alcools qui présentent un ou plusieurs groupes hydroxyle estérifiables,
caractérisé en ce qu'
on utilise comme réacteur de transestérification de 0,01 à 10 % en poids, par rapport à l'ensemble du mélange réactionnel, de catalyseurs mixtes, constitués de :
5 à 95 % molaire, par rapport au catalyseur mixte, d'oxydes de diorganoétain de formule II : 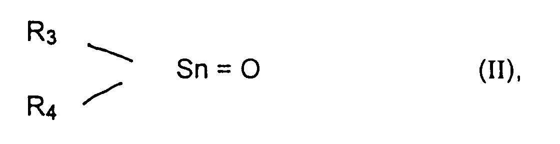 dans laquelle
R₃ et R₄ représentent des radicaux aliphatiques, aromatiques ou araliphatiques ayant de 1 à 12 atomes de carbone,
ainsi que de
95 à 5 % molaire, par rapport au catalyseur mixte, d'halogénures d'organoétain de formule IIIa et/ou de formule IIIb : 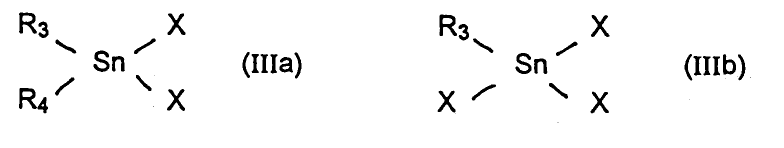 dans laquelle
R₃ et R₄ représentent à nouveau des radicaux aliphatiques, aromatiques ou araliphatiques comportant de 1 à 12 atomes de carbone, et X représente Cl, Br, CN, NCO ou NCS,
et en ce que
le catalyseur mixte après la fin de la transestérification est séparé par addition d'eau à des quantités de 10 à 150 parties en poids par rapport à 100 parties en poids de l'ensemble du mélange réactionnel.

2. Procédé selon la revendication 1,
caractérisé en ce que
les catalyseurs mixtes sont utilisés à des quantités de 0,1 à 5 % en poids par rapport à l'ensemble du mélange réactionnel.

3. Procédé selon les revendications 1 et 2,
caractérisé en ce que
les catalyseurs mixtes se composent de 25 à 75 % molaire d'oxydes de diorganoétain de formule II, et de 75 à 25 % molaire d'halogénures d'organoétain de formule IIIa et/ou de formule IIIb.

4. Procédé selon les revendications 1 à 3,
caractérisé en ce que
les alcools utilisés dans la transestérification présentent deux ou plusieurs groupes hydroxyle estérifiables.

5. Procédé selon la revendication 4,
caractérisé en ce que
les alcools ayant plusieurs groupes hydroxyle estérifiables sont choisis dans le groupe des :
alcools bifonctionnels de formule IV :
HO-R'-OH (IV),
dans laquelle
R' représente un radical aliphatique ou araliphatique éventuellement ramifié et comportant de 2 à 24 atomes de carbone,
alcools trifonctionnels de formule V : 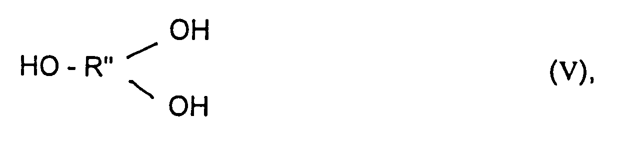 dans laquelle
R'' représente un radical aliphatique ou araliphatique éventuellement ramifié et comportant de 3 à 30 atomes de carbone,
ainsi que des alcools tétrafonctionnels de formule VI : 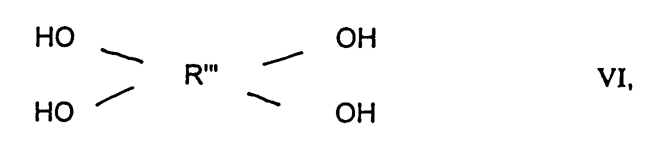 dans laquelle
R''' représente un radical aliphatique ou araliphatique éventuellement ramifié et comportant de 4 à 40 atomes de carbone

6. Procédé selon les revendications 1 à 5,
caractérisées en ce qu'
on utilise les esters d'acide (méth)acrylique de formule I à des quantités de 1,2 à 15 moles par mole de groupes hydroxyle estérifiables.

7. Procédé selon les revendications 1 à 6,
caractérisé en ce qu'
on conduit la réaction de transestérification à des températures comprises entre 50 et 130°C.
